# EUROPEAN PATENT APPLICATION

(11) **EP 4 361 270 A1**
(43) Date of publication of application: **01.05.2024**
(21) Application number: 22828816.3
(22) Date of filing: 24.06.2022
(51) Int. Cl.: C12N 15/113, C12N 15/63, A61K 48/00

(54) **NON-NATURAL 5'-UNTRANSLATED REGION AND 3'-UNTRANSLATED REGION AND USE THEREOF**

(30) Priority: 24.06.2021 KR 20210082600; 22.12.2021 KR 20210185375
(71) Applicant: Hanmi Pharm. Co., Ltd., Hwaseong-si, Gyeonggi-do 18536 (KR)
(72) Inventor: HAN, Seung Su, Hwaseong-si, Gyeonggi-do 18469 (KR); PARK, Da Hyeon, Hwaseong-si, Gyeonggi-do 18469 (KR); OH, Euh Lim, Hwaseong-si, Gyeonggi-do 18469 (KR); HEO, Yong Ho, Hwaseong-si, Gyeonggi-do 18469 (KR); LEE, Jin Bong, Hwaseong-si, Gyeonggi-do 18469 (KR); DONG, Joo Young, Hwaseong-si, Gyeonggi-do 18469 (KR); SHIN, Seung Hyun, Hwaseong-si, Gyeonggi-do 18469 (KR); LIM, Chang Gyu, Hwaseong-si, Gyeonggi-do 18469 (KR)
(74) Representative: Brevalex
(86) International application number: PCT/KR2022/009020
(87) International publication number: WO 2022/270969

(57) **Abstract**

Provided is an isolated polynucleotide including a nucleotide sequence coding for a non-natural 5'-untranslated region (5'-UTR) and a nucleotide sequence coding for a non-natural or natural 3'-untranslated region (3'-UTR). As such, the polynucleotide increases mRNA stabilization and translation efficiency and thus can be used for effectively acquiring a desired polypeptide.

## Description

### Technical Field

The disclosure relates to a non-naturally occurring 5'-untranslated region and a 3'-untranslated region and a use thereof.

### Background Art

Nucleic acid vaccines use antigens coded by DNA or RNA. DNA vaccines generally include antigen-coding gene inserted into a bacterial plasmid, which is regulated by a eukaryotic promoter. On the other hand, RNA vaccines use a messenger RNA (mRNA) or other antigen-coding RNAs. Similar to protein vaccines, nucleic acid vaccines may be delivered via various routes such as intramuscular, subcutaneous, mucosal, or percutaneous routes.

DNA vaccines are known to induce weaker immune response than peptide vaccines, cell vaccines, viral vector vaccines and RNA vaccines. In addition to having low immunogenicity, DNA vaccines may be inserted into the host genome to induce oncogenesis. As a result, there is a need for RNA-based vaccines.

### Disclosure

### Technical Field

An aspect provides an isolated polynucleotide including a nucleotide sequence encoding a non-naturally occurring 5'-untranslated region (5'-UTR), a nucleotide sequence encoding a 3'-untranslated region (3'-UTR), or a combination thereof.

Another aspect provides RNA that may be obtainable by transcription using the polynucleotide of the first aspect as a template.

Another aspect provides an isolated polynucleotide including a non-naturally occurring 5'-untranslated region (5'-UTR) nucleotide sequence, a 3'-untranslated region (3'-UTR) nucleotide sequence, or a combination thereof.

Another aspect provides a method of obtaining RNA, including transcribing RNA with the polynucleotide as a template.

Another aspect provides a method of obtaining a polypeptide, including translating the RNA obtained by the above-described method.

Another aspect provides a composition including the polynucleotide for delivering the polynucleotide to a subject.

Another aspect provides a composition including the polynucleotide for inducing an immune response to the polynucleotide.

Another aspect provides a method of using the polynucleotide including introducing the polynucleotide into a host cell.

### Technical solution

As used herein, the term "identity", used in relation to polypeptides or polynucleotides, refers to relation between at least two sequences of polypeptides or polynucleotides determined by comparing the sequences. The identity indicates a level of sequence relatedness determined by a number of matchings between strings of amino acid residues or nucleotide residues. Identity of related polypeptides or polynucleotides may be calculated by a known method. The term "% Identity", when applied to polypeptides or polynucleotides, is defined as a percentage of residues in a candidate sequence identical to residues of a second sequence after aligning the candidate amino acid sequence or nucleotide sequence with the second sequence in order to obtain the maximum percentage identity, and introducing a gap when needed. Methods and programs for the alignment is known in the art. The program may be, for example, BLAST, Smith-Waterman algorithm, or Needleman-Wunsch algorithm.

As used herein, "5'-untranslated region (5'-UTR)" refers to an mRNA region that does not encode a polypeptide, directly upstream (i.e., 5') from the first codon of the mRNA transcript to be translated by ribosomes, that is, an initiation codon.

As used herein, "3'-untranslated region (3'-UTR)" refers to an mRNA region that does not encode a polypeptide, directly downstream (i.e., 3') from the codon of the mRNA transcript signaling termination of translation, that is, a stop codon.

As used herein, "open reading frame (ORF)" refers to a continuous region of DNA that encodes a polypeptide, beginning from an initiation codon, for example, a methionine codon (ATG), and ending with a stop codon, such as TAA, TAG, or TGA.

As used herein, "polyadenylate sequence," or "poly (A) tail" refers to an mRNA region including multiple continuous adenosine monophosphates downstream from a 3'-UTR, for example, directly downstream (i.e., 3') from a 3'-UTR. The poly (A) tail may include 10 to 300 adenosine monophosphates. For example, the poly (A) tail may include 10, 20, 30, 40, 50, 60, 70, 80, 90, 100, 110, 120, 130, 140, 150, 160, 170, 190, 200, 210, 220, 230, 240, 250, 260, 270, 280, 290, or 300 adenosine monophosphates. The poly (A) tail may include, for example, 50 to 250 adenosine monophosphates. The poly (A) tail in a living body, such as a cell or a subject, may serve to protect the mRNA from an attack of an enzyme, for example, an enzyme in the cytoplasm, and aid in termination of transcription, export of the mRNA from the nucleus, and translation.

As used herein, the term "operatively linked" refers to connection of nucleotide sequences on a single nucleic acid fragment so that a function is affected by another. For example, a promoter is operatively linked to a coding sequence (for example, ORF) when the promoter is capable of affecting the expression of the coding sequence (that is, when transcription of the coding sequence is regulated by the promoter). The coding sequence may be operatively linked to a regulatory sequence in a sense or anti-sense direction.

As used herein, unless a location of a nucleotide sequence is otherwise specified, a nucleotide sequence is connected or located from the 5'-end to the 3'-end.

As used herein, the term "vector" or "nucleic acid construct" refers to an arbitrary nucleic acid that may carry a gene, ORF, or a DNA fragment into a cell. The vector may be, for example, one which may be replicated in a cell. The vector may be a virus, a bacteriophage, a provirus, a plasmid, a phagemid, a transposon, or artificial chromosomes such as an yeast artificial chromosome (YAC), a bacterial artificial chromosome (BAC), or a plant artificial chromosome (PLAC).

The first aspect provides an isolated polynucleotide including a nucleotide sequence encoding a non-naturally occurring 5'-untranslated region (5'-UTR), a nucleotide sequence encoding 3'-untranslated region (3'-UTR), or a combination thereof.

The isolated polynucleotide may include a nucleotide sequence encoding a non-naturally occurring 5'-UTR and a nucleotide sequence encoding a non-naturally occurring 3'-UTR. The isolated polynucleotide may include a nucleotide sequence encoding a non-naturally occurring 5'-UTR and a nucleotide sequence encoding a naturally occurring 3'-UTR.

The isolated polynucleotide may be a polydeoxyribonucleotide.

The 5'-UTR may include a non-naturally occurring nucleotide sequence. The 5'-UTR may have sequence identity to a nucleotide sequence of SEQ ID NO: 1 of 70 % or more, for example, 80 % or more, 90 % or more, or 95 % or more. The 5'-UTR may include a sequence of SEQ ID NO: 1, 2, 3, 4, 5, 6, 7, 8, 9, 10, 11, 12, or a combination thereof. The 5'-UTRs may be polyribonucleotides. A nucleotide sequence encoding the non-naturally occurring 5'-untranslated region (5'-UTR) may include a sequence of SEQ ID NO: 22, 23, 24, 25, 26, 27, 28, 29, 30, 31, 32, 33, or a combination thereof. These sequences may be polydeoxyribonucleotides.

The 3'-UTR may include a non-naturally occurring or a naturally occurring nucleotide sequence. The 3'-UTR may have sequence identity of 70 % or more, for example, 80 % or more, 90 % or more, or 95 % or more to a nucleotide sequence of SEQ ID NO: 15, and may have sequence identity of 70 % or more, for example, 80 % or more, 90 % or more, or 95 % or more to a nucleotide sequence of SEQ ID NO: 20. The 3'-UTR may include a sequence of SEQ ID NO: 14, 15, 16, 17, 18, 19, 20, or a combination thereof. A nucleotide sequence encoding the non-naturally occurring, or naturally occurring 3'-untranslated region (3'-UTR) may include SEQ ID NOS: 34, 35, 36, 37, 38, 39, 40, or a combination thereof.

A nucleotide sequence encoding the non-naturally occurring, or naturally occurring 3'-UTR may further include a recognition site for a restriction enzyme. The restriction enzyme may include Xhol, Nhel, or Xhol and Nhel.

The nucleotide sequence encoding the 5'-UTR may further include a nucleotide sequence of an upstream promoter region operatively linked to the 5'-UTR.

The nucleotide sequence encoding the 5'-UTR may include a transcribable nucleotide sequence or a nucleotide sequence for introducing the transcribable nucleotide sequence, and the transcribable nucleotide sequence or the nucleotide sequence for introducing the transcribable nucleotide sequence may be operatively linked to the 5'-UTR, downstream of the nucleotide encoding the 5'-UTR.

The transcribable nucleotide sequence may be a sequence encoding a polypeptide or RNA. The polypeptide may be an antigenic or therapeutic polypeptide. The antigenic polypeptide may be a viral antigenic polypeptide. The viral antigenic polypeptide may be at least one betacoronavirus (BetaCoV) antigenic polypeptide, at least one respiratory syncytial virus (RSV) antigenic polypeptide, at least one measles virus (MeV) antigenic polypeptide, at least one human metapneumovirus (hMPV) antigenic polypeptide, at least one human parainfluenza virus (PIV) antigenic polypeptide, an antigenic fragment thereof, or a combination thereof. The BetaCoV may be MERS-CoV, SARS-CoV, SARS-CoV-2, HCoV-OC43, HCoV-229E, HCoV-NL63, HCoV-NL, HCoV-NH, HCoV-HKU1, a variant thereof, or a combination thereof. SARS-CoV-2 is identified in 2019, and is also known as COVID-19. The antigenic polypeptide may be a spike protein of BetaCoV or an antigenic fragment thereof. The therapeutic polypeptide may be, for example, an antibody, a hormone, a cytokine, an enzyme, a derivative thereof, or a combination thereof.

The nucleotide sequence for introducing the transcribable nucleotide sequence may be a cloning site. The cloning site may be a multiple cloning site. The cloning site may include a recognition site of a restriction enzyme, a cleavage site, or a combination thereof.

The nucleotide sequence encoding the 5'-UTR, and transcribable nucleotide, or nucleotide sequence for introducing the transcribable nucleotide may be linked to form a common transcript when transcribed in vitro or in vivo.

The promoter, nucleotide sequence encoding the 5'-UTR, transcribable nucleotide, or nucleotide sequence for introducing the transcribable nucleotide may be linked to form a common transcript when transcribed in vitro or in vivo.

The nucleotide sequence encoding the 3'-UTR may further include a nucleotide sequence of a downstream polyadenylate or a polyadenylate attachment signal (poly (A) attachment signal) operably linked to the 3'-UTR. The nucleotide sequence encoding the 3'-UTR may include a transcribable nucleotide sequence or a nucleotide sequence for introducing the transcribable nucleotide sequence, and the transcribable nucleotide sequence or the nucleotide sequence for introducing the transcribable nucleotide sequence may be operatively linked to the 3'-UTR, upstream of the nucleotide encoding the 3'-UTR.

The transcribable nucleotide, or nucleotide sequence for introducing the transcribable nucleotide, nucleotide sequence encoding the 3'-UTR, and polyadenylate or polyadenylate attachment signal may be linked so as to form a common transcript when transcribed in vitro or in vivo, or to add a poly (A) tail downstream of the 3'-UTR of the common transcript.

In the polynucleotide, the nucleotide sequence encoding the 5'-UTR may include a transcribable nucleotide sequence or nucleotide sequence for introducing the transcribable nucleotide sequence operatively linked to the 5'-UTR sequence. Downstream of the 5'-UTR sequence, the transcribable nucleotide sequence or nucleotide sequence for introducing the transcribable nucleotide sequence may be operatively linked to the 5'-UTR sequence, and the nucleotide sequence encoding the 3'-UTR may include a nucleotide sequence of a downstream polyadenylate or polyadenylate attachment signal operatively linked to the 3'-UTR and may be operatively linked to the transcribable nucleotide sequence or the nucleotide sequence for introducing the transcribable nucleotide sequence, upstream of the 3'-UTR sequence. For example, the polynucleotide may be one, in which are sequentially connected: a nucleotide sequence encoding the 5'-UTR, a nucleotide sequence encoding a polypeptide or a cloning site nucleotide sequence, a nucleotide sequence encoding the 3'-UTR, and a polyadenylate or polyadenylate attachment signal nucleotide sequence. In the polynucleotide, the nucleotide sequence encoding the 5'-UTR may include a nucleotide sequence of SEQ ID NO: 22 or 31, the nucleotide sequence encoding the 3'-UTR may include a nucleotide sequence of SEQ ID NO: 40, and the polyadenylate nucleotide sequence may include a nucleotide sequence of SEQ ID NO: 41.

The nucleotide sequence encoding the 5'-UTR may include a nucleotide sequence of an upstream promoter region operatively linked to the 5'-UTR, a transcribable nucleotide sequence or a nucleotide sequence for introducing the transcribable nucleotide sequence operatively linked to the promoter; the transcribable nucleotide sequence or nucleotide sequence for introducing the transcribable nucleotide sequence is operatively linked to the 5'-UTR sequence, downstream of the 5'-UTR sequence; the nucleotide sequence encoding the 3'-UTR may include a nucleotide sequence of a downstream polyadenylate or polyadenylate attachment signal operatively linked to the 3'-UTR, and may be operatively linked to the transcribable nucleotide sequence or the nucleotide sequence for introducing the transcribable nucleotide sequence, upstream of the 3'-UTR sequence. For example, the polynucleotide may be one, in which are sequentially connected: a promoter, a nucleotide sequence encoding the 5'-UTR, a nucleotide sequence encoding a polypeptide or a cloning site nucleotide sequence, a nucleotide sequence encoding the 3'-UTR, and a polyadenylate or polyadenylate attachment signal nucleotide sequence. In the polynucleotide, the nucleotide sequence encoding the 5'-UTR may include a nucleotide sequence of SEQ ID NO: 22 or 31, the nucleotide sequence encoding the 3'-UTR may include a nucleotide sequence of SEQ ID NO: 40, and the polyadenylate nucleotide sequence may include a nucleotide sequence of SEQ ID NO: 41

The nucleotide sequence encoding the 5'-UTR, nucleotide sequence encoding a polypeptide or cloning site nucleotide sequence, nucleotide sequence encoding the 3'-UTR, and polyadenylate or polyadenylate attachment signal may be linked so as to form a common transcript when transcribed in vitro or in vivo, or to add a poly (A) tail downstream of the 3'-UTR of the common transcript.

The promoter, nucleotide sequence encoding the 5'-UTR, nucleotide sequence encoding a polypeptide or cloning site nucleotide sequence, nucleotide sequence encoding the 3'-UTR, and polyadenylate or polyadenylate attachment signal may be linked so as to form a common transcript when transcribed in vitro or in vivo, or to add a poly (A) tail downstream of the 3'-UTR of the common transcript.

The polynucleotide may be a nucleic acid construct or a vector. As used herein, "polyadenylate attachment signal" refers to a nucleotide sequence that aids in adding a polyadenylate to an immature mRNA. The nucleic acid construct or vector may include a regulatory sequence needed for expressing the polynucleotide. The expression may be transcription, translation, or a combination thereof. The nucleic acid construct or vector may include expression construct or vector.

The second aspect provides RNA that may be obtainable by transcription using the polynucleotide of the first aspect as a template. The RNA may be an mRNA. The mRNA may include a signal sequence. The mRNA may comprise any one nucleotide sequence selected from SEQ ID NOS: 63 to 76. The mRNA may be one in which at least one U is substituted by an N1-methyl-pseudouridine in the sequence. In the mRNA, GA at the 5'-end may have a cap structure of m7(3'OMeG)(5')ppp(5')(2'OMeA). In the mRNA, all Us may be substituted by N1-methyl-pseudouridines. The mRNA may be one, in which all Us are substituted by N1-methyl-pseudouridines, and GA at the 5'-end has a cap structure of m7(3'OMeG)(5')ppp(5')(2'OMeA).

The third aspect provides a isolated polynucleotide including a non-naturally occurring 5'-untranslated region (5'-UTR) nucleotide sequence, a non-naturally occurring or naturally occurring 3'-untranslated region (3'-UTR) nucleotide sequence, or a combination thereof.

In the polynucleotide of the third aspect, the 5'-UTR may have sequence identity of 70 % or more, for example, 80 % or more, 90 % or more, or 95 % or more to a nucleotide sequence of SEQ ID NO: 1. The 5'-UTR may include a sequence of SEQ ID NO: 1, 2, 3, 4, 5, 6, 7, 8, 9, 10, 11, 12, or a combination thereof. A nucleotide sequence encoding the non-naturally occurring 5'-untranslated region (5'-UTR) may include a sequence of SEQ ID NO: 22, 23, 24, 25, 26, 27, 28, 29, 30, 31, 32, 33, or a combination thereof.

The 3'-UTR may be one having sequence identity to a nucleotide sequence of SEQ ID NO: 15 of 70 % or more, for example, 80 % or more, 90 % or more, or 95 % or more, and having sequence identity to a nucleotide sequence of SEQ ID NO: 20 of 70 % or more, for example, 80 % or more, 90 % or more, or 95 % or more. The 3'-UTR may include a sequence of SEQ ID NO: 14, 15, 16, 17, 18, 19, 20, or a combination thereof. A nucleotide sequence encoding the non-naturally occurring, or naturally occurring 3'-untranslated region (3'-UTR) may include a sequence of SEQ ID NO: 34, 35, 36, 37, 38, 39, 40, or a combination thereof.

The polynucleotide of the third aspect may include a non-naturally occurring 5'-untranslated region (5'-UTR) and a naturally occurring a 3'-untranslated region (3'-UTR).

The 5'-UTR nucleotide sequence may include a nucleotide sequence encoding a polypeptide, and the nucleotide sequence encoding a polypeptide may be operatively linked to the 5'-UTR, downstream of the 5'-UTR nucleotide. The polypeptide may be an antigenic or therapeutic polypeptide. The antigenic polypeptide may be a viral antigenic polypeptide. The viral antigenic polypeptide may be at least one BetaCoV antigenic polypeptide, at least one RSV antigenic polypeptide, at least one MeV antigenic polypeptide, at least one hMPV antigenic polypeptide, at least one hPIV antigenic polypeptide, an antigenic fragment thereof, or a combination thereof. The BetaCoV may be MERS-CoV, SARS-CoV, SARS-CoV-2, HCoV-OC43, HCoV-229E, HCoV-NL63, HCoV-NL, HCoV-NH, HCoV-HKU1, a variant thereof, or a combination thereof. The antigenic polypeptide may be a spike protein of BetaCoV or an antigenic fragment thereof. The spike protein of the BetaCoV may be a natural spike protein of SARS-CoV-2 or a variant thereof. The variant includes alpha, beta, gamma, delta, epsilon, and omicron variants. The natural spike protein of SARS-CoV-2 or spike proteins of alpha, beta, gamma, delta, epsilon, and omicron variants of SARS-CoV-2 may comprise an amino acid sequence of SEQ ID NOS: 42 to 48, respectively. The therapeutic polypeptide may be, for example, an antibody, a hormone, a cytokine, an enzyme, a derivative thereof, or a combination thereof.

The 5'-UTR nucleotide sequence, and the nucleotide sequence encoding a polypeptide may be connected in a common transcript.

The 3'-UTR nucleotide may further include a downstream polyadenylate nucleotide sequence operatively linked thereto. The polyadenylate nucleotide sequence may include, for example, 20 or more, 40 or more, 80 or more, or 100 or more, and 500 or less, 400 or less, 300 or less, 200 or less, or 150 or less adenyl nucleotides.

The 3'-UTR nucleotide sequence may include a nucleotide sequence encoding a polypeptide, and the nucleotide sequence encoding a polypeptide may be operatively linked to the 3'-UTR, upstream of the 3'-UTR nucleotide sequence. The nucleotide sequence encoding the polypeptide may be a ribonucleotide sequence, for example, an mRNA.

The nucleotide sequence encoding the polypeptide and the 3'-UTR nucleotide sequence may be connected in a common transcript.

The 5'-UTR may include a nucleotide sequence encoding a polypeptide, and the nucleotide sequence encoding a polypeptide may be operatively linked to the 5'-UTR sequence, downstream of the 5'-UTR sequence, and the 3'-UTR may include a downstream polyadenylate nucleotide sequence operatively linked to the 3'-UTR and may be linked to the nucleotide sequence encoding a polypeptide, upstream of the 3'-UTR sequence.

The 5'-UTR nucleotide sequence, nucleotide sequence encoding a polypeptide, 3'-UTR nucleotide sequence, and poly (A) tail may be connected in a common transcript.

The 5'-UTR and the 3'-UTR may have an activity of increasing translation efficiency, stability of the nucleotide sequence encoding a polypeptide, or a combination thereof, compared to a case where a naturally occurring 5'-UTR and/or naturally occurring 3'-UTR is used.

The polynucleotide may be RNA, for example, an mRNA. The polynucleotide may include a 5'-cap. The term "5'-cap" refers to a cap structure found at the 5'-end of an mRNA molecule. The 5'-cap may include a guanosine nucleotide connected to an mRNA through an unusual triphosphate linkage to the 5'-end. The guanosine may be methylated at the 7-position, and is for example, m⁷G, or 3'-O-Me-m⁷G. The term "conventional 5'-cap" indicates a naturally occurring RNA 5'-cap, for example, 7-methylguanosine (m7G). As used herein, the term "5'-cap" includes a 5'-cap analog, which resembles an RNA-cap structure and is attached to RNA, and is, for example, modified to have the ability to stabilize the RNA in vivo and/or in a cell. A 5'-cap or a 5'-cap analog may be provided to RNA by transcribing a DNA template in a presence of 5'-caps or 5'-cap analogs and by co-transcriptionally inserting the 5'-cap or the 5'-cap analog into the RNA strand being generated, or by using a post-transcriptional capping enzyme, for example, a capping enzyme of a vaccinia virus to generate 5'-caps or 5'-cap analogs. The 5'-end cap structure may be, for example, 7mG(5')ppp(5')lmpNp, 3'-O-Me-m7G(5')ppp(5')G, m7(3'OMeG)(5')ppp(5')(2'OMeA) or m7G(5')ppp(5')(2'OMeA)pG.

The polynucleotide may be RNA, the 5'-UTR nucleotide sequence may comprise a nucleotide sequence of SEQ ID NO: 1 or 10, the 3'-UTR nucleotide sequence may comprise a nucleotide sequence of SEQ ID NO: 20, and the polyadenylate nucleotide sequence may comprise a nucleotide sequence of SEQ ID NO: 101. The 3'-UTR may further include a recognition sequence for a restriction enzyme. The restriction enzyme may include Xhol, Nhel, or Xhol and Nhel.

In the polynucleotide, at least one U may be substituted by an N1-methyl-pseudouridine.

The polynucleotide may comprise any one nucleotide sequence selected from SEQ ID NOS: 63 to 76, and at least one U in the polynucleotide may be substituted by an N1-methyl-pseudouridine. In the polynucleotide, every U may be substituted by an N1-methyl-pseudouridine.

In addition, the polynucleotide may have a cap structure of m7(3'OMeG)(5')ppp(5')(2'OMeA) at the 5'-end.

The fourth aspect provides a method of obtaining RNA, including transcribing RNA with the polynucleotide of the first aspect as a template. The transcription may include transcription in vitro, ex vivo, or in vivo. The in vivo transcription may include a transcription in a subject. The RNA may be an mRNA. The mRNA may comprise any one nucleotide sequence selected from SEQ ID NOS: 63 to 76. The mRNA may be one in which at least one U is substituted by an N1-methyl-pseudouridine in the sequence. In the mRNA, GA at the 5'-end may have a cap structure of m7(3'OMeG)(5')ppp(5')(2'OMeA). In the mRNA, all Us may be substituted by N1-methyl-pseudouridines. The mRNA may be one, in which all Us are substituted by N1-methyl-pseudouridines, and GA at the 5'-end has a cap structure of 5'-[1,2-[(3'-O-methyl)m7G-(5'→5')-ppp-Am]]. In the structure, Am indicates 2'-O-methyladenosine. However, the 5'-cap structure of the mRNA is not limited to this structure, and may be substituted by a naturally occurring 5'-cap structure or a 5'-cap analog known in the art.

The term "in vitro transcription" or "RNA in vitro transcription" refers to a process of synthesizing RNA including an mRNA in a non-cell system, that is, in vitro. A cloning vector DNA including a plasmid DNA vector may be applied as a template for generating an RNA transcript. These cloning vectors are also generally known as transcription vectors. RNA may be obtained by DNA-dependent in vitro transcription with an appropriate DNA template. The DNA template may be a linearized plasmid DNA template. The promoter regulating RNA in vitro transcription may be an arbitrary promoter for a DNA-dependent RNA polymerase. The DNA-dependent RNA polymerase may be a T7 RNA polymerase, a T3 RNA polymerase, a SP6 RNA polymerase, or a combination thereof. The DNA template for RNA in vitro transcription may be obtained by cloning a nucleic acid and introducing the same into a vector for RNA in vitro transcription. The DNA may include cDNA corresponding to each RNA to be transcribed in vitro. The vector for RNA in vitro transcription may be circular plasmid DNA. The cDNA may be obtained by reverse-transcription of an mRNA or by a chemical synthesis.

The transcription vector may include a promoter, a nucleotide sequence encoding the 5'-UTR, a sequence encoding a polypeptide, for example, an open reading frame (ORF), a nucleotide sequence encoding the 3'-UTR, and a poly (A) tail. The nucleotide sequence encoding the 5'-UTR may comprise at least one selected from nucleotide sequences of SEQ ID NOS: 22 to 33. The nucleotide sequence encoding the 3'-UTR may comprise at least one selected from nucleotide sequences of SEQ ID NOS: 34 to 40. The transcription vector may also include an E. coli replication origin ColE1 ori, a selection marker gene, or a combination thereof. The promoter may be a T7 promoter. The selection marker may be an antibiotic resistance enzyme, for example, a kanamycin resistance enzyme.

The in vitro transcription may include obtaining transformed host cells by introducing the transcription vector into host cells, for example, E. coli, and proliferating the host cells containing the plasmid DNA, which becomes a template for an mRNA, by culturing the host cells. The in vitro transcription may include isolating the plasmid DNA from the proliferated host cells, for example, E. coli. The isolation of the plasmid DNA may include isolating cells from the culture and isolating the plasmid DNA from the cells. The isolation of the cells may be performed by centrifugation, separation, precipitation, or a combination thereof. Isolation of plasmid DNA from cells may be performed by the alkali extraction method, affinity chromatography, high performance liquid chromatography (HPLC), electrophoresis, or a combination thereof.

The method of obtaining RNA may include producing RNA transcripts of the polynucleotide by culturing the cells including the polynucleotide of the first aspect. The RNA transcript may be an mRNA. The mRNA may comprise any one nucleotide sequence selected from SEQ ID NOS: 63 to 76. The mRNA may be one in which at least one U is substituted by an N1-methyl-pseudouridine in the sequence. In the mRNA, GA at the 5'-end may have a cap structure of 5'-[1,2-[(3'-O-methyl)m7G-(5'→5')-ppp-Am]]. In the mRNA, all Us may be substituted by N1-methyl-pseudouridines. In the mRNA, all Us may be substituted by N1-methyl-pseudouridines, and GA at the 5'-end may have a cap structure of 5'-[1,2-[(3'-O-methyl)m7G-(5'→5')-ppp-Am]]. However, the 5'-cap structure of the mRNA is not limited to this structure, and may be substituted by a naturally occurring 5'-cap structure or a 5'-cap analog known in the art.

The fifth aspect provides a method of obtaining a polypeptide including translating the RNA of the third aspect, or the RNA obtained by the method of the fourth aspect. The RNA of the third aspect or the RNA obtained by the method of the fourth aspect may be, for example, an mRNA.

The method may include producing the polypeptide by culturing cells including the polynucleotide of the first aspect.

The sixth aspect provides a composition for introducing the polypeptide including the polynucleotide of the third aspect into a subject. The polynucleotide of the third aspect may be RNA, for example, an mRNA. The polynucleotide of the third aspect may be RNA obtained by the method of the fourth aspect.

The seventh aspect provides a composition for inducing an immune response to the polypeptide including the polynucleotide of the third aspect as an active ingredient. The polynucleotide of the third aspect may be RNA, for example, an mRNA. The polynucleotide of the third aspect may be RNA obtained by the method of the fourth aspect.

In the sixth aspect and the seventh aspect, the composition may be for providing immunogenicity or for treating a disease. The disease may differ according to the selected polypeptide. The disease may be, for example, infection caused by a virus or a foreign cell, a cancer, a metabolic disease, an inflammatory disease, a gastrointestinal disease, an endocrine disease, sepsis, or an autoimmune disease. The disease may be caused by enzyme deficiency, as in the case of Hunter syndrome, Gaucher disease, and Fabry disease, and may be treated with the enzyme replacement therapy. In the disease, the foreign cells may be prokaryotic or eukaryotic cells. The foreign cells may be bacteria.

The polynucleotide may include a nucleotide sequence encoding the polypeptide. The polypeptide may be an antigenic or therapeutic polypeptide. The antigenic polypeptide may be a viral antigenic polypeptide. The viral antigenic polypeptide may be at least one BetaCoV antigenic polypeptide, at least one RSV antigenic polypeptide, at least one MeV antigenic polypeptide, at least one hMPV antigenic polypeptide, at least one hPIV antigenic polypeptide, an antigenic fragment thereof, or a combination thereof. The BetaCoV may be MERS-CoV, SARS-CoV, SARS-CoV-2, HCoV-OC43, HCoV-229E, HCoV-NL63, HCoV-NL, HCoV-NH, HCoV-HKU1, a variant thereof, or a combination thereof. The antigenic polypeptide may be a spike protein of BetaCoV or an antigenic fragment thereof. The therapeutic polypeptide may be, for example, an antibody, a hormone, a cytokine, an enzyme, a derivative thereof, or a combination thereof. The composition may be for preventing, treating, or diagnosing a symptom, or an infection in a subject. The subject may be a mammal, including a human. The infection may be a bacterial or a viral infection. The virus may be BetaCoV, RSV, MeV, hMPV, or hPIV. The BetaCoV may be MERS-CoV, SARS-CoV, SARS-CoV-2, HCoV-OC43, HCoV-229E, HCoV-NL63, HCoV-NL, HCoV-NH, HCoV-HKU1, a variant thereof, or a combination thereof. The composition may be used for priming immune effector cells. For example, the composition may be used to activate peripheral blood mononuclear cells (PBMCs) ex vivo, and to infuse into a subject. The composition may be used to induce immune response to SARS-CoV-2 or a variant thereof. The composition may be used to prevent infection by SARS-CoV-2 or a variant thereof.

The composition may be injected into a subject and the RNA polynucleotide (for example, an mRNA) may be translated in vivo to produce antigenic polypeptides.

An "effective amount" of the composition including a polynucleotide having at least one translatable region encoding an antigenic polypeptide may be contacted with a cell, a tissue, or a subject.

The effective amount may be determined based on the target tissue, target cell, means of administration, physical properties of the polynucleotide (for example, size of the polynucleotide and amount of modified nucleoside in the polynucleotide) and other components of the composition. Generally, an effective amount of the composition may cause an induced or boosted immune response as a function of the antigen production in the cells.

The composition may be administered with other preventive or therapeutic compounds. The preventive or therapeutic compound may be, for example, an adjuvant or a booster. As used herein, when referring to a preventive composition, such as a vaccine, the term "booster" refers to additional administration of the preventive composition. A booster or a booster vaccine may be given after an early administration of the preventive composition. The time between the initial administration of the preventive composition and the administration of the booster may be 1 week, 2 weeks, 3 weeks, 1 month, 2 months, 3 months, 6 months or 1 year. In addition, the composition may not include an adjuvant.

The composition may be administered via an intramuscular route, a subcutaneous route, an intradermal route, an intranasal route, or a pulmonary route.

The composition may include at least one pharmaceutically acceptable excipient. The polynucleotide in the composition may be formulated with the excipient or may be made into a complex with the excipient. The excipient may be one known in the art.

As used herein, the term "active ingredient" refers to the composition or the polynucleotide included therein, for example, an RNA polynucleotide including an mRNA. The RNA polynucleotide may be, for example, encoding an antigenic or therapeutic polypeptide.

Relative amounts of active ingredients included in the composition, pharmaceutically acceptable excipients, and/or other additional components may vary depending on the identity and size of the subject to be treated, and/or conditions and routes of the administration. For example, the composition may include 0.1 % to 100 %, for example, 0.5 % to 50 %, 1.0 %to 30 %, 5.0 % to 80 %, or 80 %(w/w) or more active ingredients.

In the composition, the polynucleotide may be an mRNA. The mRNA may include a stabilizing element including the 5'-UTR, and/or 3'-UTR. The mRNA may further include additional structures such as a 5'-cap structure or a 3'-poly (A) tail. 5'-UTR and 3'-UTR are generally transcribed from the genomic DNA and are elements of an immature mRNA. Structural features of a mature mRNA, such as a 5'-cap and a 3'-poly (A) tail are generally added to the transcribed (immature) mRNA during an mRNA processing. The poly (A) tail is a stretch of adenine nucleotides generally added to the transcribed mRNA. The poly (A) tail may include, for example, 400 or less adenine nucleotides. In some embodiments, a length of the 3'-poly (A) tail may be an essential element in the stability of an individual mRNA.

The composition may be formulated as nanoparticles. The nanoparticles may be for delivering polynucleotides such as an mRNA into cells, and is known in the art. For example, the nanoparticles may be known nanoparticles used in a vaccine, for example, in RNA vaccines, to induce an immune response to COVID-19. The nanoparticle may be a lipid nanoparticle (LNP). The composition may be formulated as lipid nanoparticles (LNPs) or may be bound to LNPs. The binding may include binding within the LNP or to the surface of the LNP. For example, the composition may be formulated inside a lipid polycation composite or may be bound to a lipid polycation composite. The lipid polycation composite is also known as a cationic lipid nanoparticle. The polycation may include MC3, Lipid 319, C12-200, 5A2-SC8, 306Oi10, Moderna Lipid 5, Acuitas A9, SM-102, ALC-0315, Arcturus Lipid 2,2 (8,8) 4C CH3, Genevant CL1, cationic polypeptides such as polylysine, polyornithine, and/or polyarginine. In addition, the composition may be formulated inside, or bound to lipid nanoparticles including non-cationic lipids, such as distearoylphosphatidylcholine (DSPC), 1,2 distearoyl-sn-glycerol-3-phosphocholine (DSPC), dipalmitoyl phosphatidylcholine (DPPC), sterols such as cholesterol, or dioleoyl phosphatidylethanolamine (DOPE). In addition, the composition may be formulated inside, or bound to lipid nanoparticles including polyethylene glycol (PEG)-lipid, such as 1,2-dimyristoyl-rac-glycero-3-methoxypolyethylene glycol-2000(PEG2000-DMG), 2-[(polyethylene glycol)-2000]-N,N-ditetradecylacetamide(ALC-0159), polyethylene glycol dimethacrylate(PEG-DMA).

Lipid nanoparticle formulations may include cationic lipids, phospholipids, sterols such as cholesterol, PEG-lipids, or combinations thereof. Lipid nanoparticle formulations may include, for example, cationic lipids, phospholipids, sterols such as cholesterol, and PEG-lipids. In addition, lipid nanoparticles may include PEG-modified lipids, non-cationic lipids, sterols, ionizable lipids, or a combination thereof. The lipid nanoparticles may include 0.5 mol% to 15 mol% of PEG-modified lipids, 5 mol% to 25 mol% of non-cationic lipids, 25 mol% to 55 mol% of sterols, and 20 mol% to 60 mol% of ionizable lipids. The PEG-modified lipid may be 1,2 dimyristoyl-sn-glycerol methoxypolyethylene glycol (PEG2000-DMG), the non-cationic lipid may be 1,2 distearoyl-sn-glycerol-3-phosphocholine (DSPC), the sterol may be cholesterol, and the ionizable cationic lipid may be Compound 1 of the structure below:

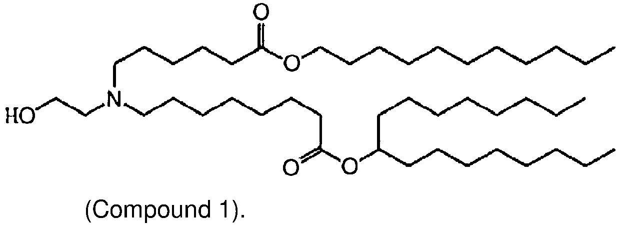

The PEG-modified lipid may be Compound 2 (ALC-0159) of the structure below, the non-cationic lipid may be 1,2 distearoyl-sn-glycerol-3-phosphocholine (DSPC), the sterol may be cholesterol, and the ionizable cationic lipid may be Compound 3 (ALC-0315) of the structure below:

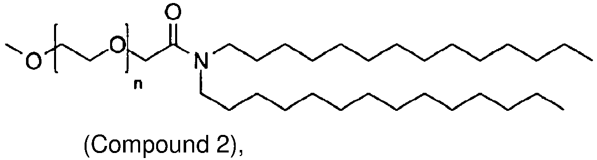

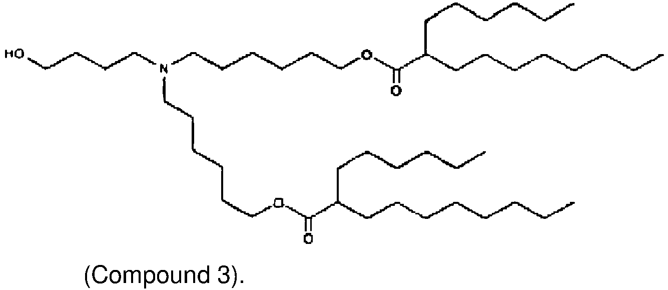

The composition may be a composition for inducing an immune response to SARS-CoV-2 or a variant thereof, including the polynucleotide, and lipid nanoparticles (LNPs) as active ingredients. The composition may be for preventing infection by SARS-CoV-2 or a variant thereof.

The eighth aspect provides a method of using the polynucleotide including introducing the polynucleotide into a host cell. The polynucleotide of the third aspect may be RNA, for example, an mRNA. The polynucleotide of the third aspect may be RNA, for example, an mRNA.

In the method, the polynucleotide may be RNA including an mRNA. The host cell may be any cell derived from a subject. The subject may be a mammal, including a human. The cell may be, for example, a stem cell, a reproductive cell or a somatic cell. In the above method, the polynucleotide may be RNA, and the host cell may be an antigen-providing cell including a dendritic cell, a monocyte or a macrophage.

The method may include injecting the polynucleotide of the third aspect into a subject. The administration may be a non-oral or oral administration. The administration may be an intramuscular, subcutaneous, intradermal, intranasal, or pulmonary administration. The subject may be a mammal, including a human.

The method may immunize a subject to the polypeptide. In addition, the method may treat a disease in a subject. The polypeptide may be an antigen protein of BetaCoV. The BetaCoV may be MERS-CoV, SARS-CoV, SARS-CoV-2, HCoV-OC43, HCoV-229E, HCoV-NL63, HCoV-NL, HCoV-NH, HCoV-HKU1, a variant thereof, or a combination thereof. The method may be inducing an immune response to BetaCoV, for example, to its spike protein, or a variant or subunit thereof, in a subject. The method may prevent infection of BetaCoV, for example, MERS-CoV, SARS-CoV, SARS-CoV-2, HCoV-OC43, HCoV-229E, HCoV-NL63, HCoV-NL, HCoV-NH, HCoV-HKU1, or variants or combinations thereof.

In the third to eighth aspects, the polynucleotide may include a 5'-end cap. The 5'-end cap may be 7mG(5')ppp(5')lmpNp, 3'-O-Me-m7G(5')ppp(5')G, or m7G(5')ppp(5')(2'OMeA)pG.

In the third to seventh aspects, the polynucleotide may include a nucleotide having at least one chemical modification. The modification may be uniformly made throughout the nucleotide. As used herein, "chemical modification" and "chemically modified" mean at least one modification of the location, pattern, percentage, or population of ribonucleotides or deoxyribonucleotides of adenosine (A), guanosine (G), uridine (U), thymine (T), or cytidine (C). In general, these terms do not refer to the modification of a ribonucleotide in the naturally occurring 5'-end an mRNA cap moiety.

A polynucleotide, for example, RNA including an mRNA may include one or more other modifications. Certain areas of a polynucleotide may include at least one modification of a nucleoside or a nucleotide. When introduced into a cell or a subject, the modified polynucleotide may show a reduced degradation in the cell or the subject, compared to an un-modified polynucleotide. In addition, when introduced into a cell or a subject, the modified polynucleotide may show a reduced immunogenicity (for example, reduced innate response) in the cell or the subject, compared to an un-modified polynucleotide.

A nucleoside having a modified cytosine may include N4-acetyl-cytidine (ac4C), 5-methyl-cytidine (m5C), 5-halo-cytidine (e.g., 5-iodo-cytidine), 5-hydroxymethyl-cytidine (hm5C), 1-methyl-pseudoisocytidine, 2-thio-cytidine (s2C), 2-thio-5-methyl-cytidine, or a combination thereof.

A nucleoside having a modified adenine may include 7-deaza-adenine, 1-methyladenosine (m1A), 2-methyl-adenine (m2A), N6-methyl-adenosine (m6A), or a combination thereof.

A nucleobase or a nucleoside having a modified guanine may include inosine (I), 1-methyl-inosine (m1!), wyosine (imG), methylwyosine (mimG), 7-deaza-guanosine, 7-cyano-7-deaza-guanosine (preQ0), 7-aminomethyl-7-deaza-guanosine (preQ1), 7-methyl-guanosine (m7G), 1-methyl-guanosine (m1G), 8-oxo-guanosine, 7-methyl-8-oxo-guanosine, or a combination thereof.

The nucleotide having a chemical modification may be pseudouridine, N1-methylpseudouridine, N1-ethylpseudouridine, 2-thiouridine, 4'-thiouridine, 5-methylcytosine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine, 2'-O-methyluridine, or a combination thereof. The chemical modification may be located at the 5th location of uracil. The chemical modification may be a modification of uracil into a N1-methylpseudouridine. The chemical modification may be a modification of uracil into a N1-ethylpseudouridine.

### Advantageous Effects

An isolated polynucleotide according to an aspect including a nucleotide sequence encoding a non-naturally occurring 5'-untranslated region (5'-UTR), a nucleotide sequence encoding a non-naturally occurring or naturally occurring 3'-untranslated region (3'-UTR), or a combination thereof, may be used to stabilize an mRNA, and thereby to increase translation efficiency.

RNA according to another aspect is relatively stable and has high translation efficiency, and therefore, may be used to translate RNA efficiently.

An isolated polynucleotide according to an aspect is relatively stable and has high translation efficiency, and therefore, may be used to translate RNA efficiently.

When a method of obtaining RNA according to another aspect is used, RNA may be obtained efficiently.

When a method of obtaining polypeptide according to another aspect is used, a polypeptide may be obtained efficiently.

An immunogenic composition according to another aspect may be used to immunize the subject efficiently.

A method of using the polynucleotide according to another aspect may be used to immunize the subject efficiently.

### Description of Drawings

FIG. 1 shows a prepared vector for producing an mRNA encoding a SARS-CoV-2 spike protein, or a variant thereof;
FIGS. 2A, 2B, and 2C show results of analyzing the produced mRNA with chromatography; and
FIG. 3 shows results of identifying expression of the spike protein by using a western blot when the produced mRNA-lipid nanoparticles (LNP) are introduced into cells.

### Best Mode

Hereinafter, the present disclosure will be described in more detail through examples. However, these examples are intended to illustrate the present disclosure, and the scope of the present disclosure is not limited to these examples.

### Example 1: 5'-UTR sequence increasing expression of gene encoding mRNA

A 5'-UTR sequence was designed that increases expression of a gene encoding an mRNA.

As a result, 12 non-naturally occurring 5'-UTR sequences having nucleotide sequences of SEQ ID NOS: 1 to 12 were obtained. The nucleotide sequence of SEQ ID NO: 2 is a sequence, in which a sequence forming a stable hairpin structure (GUCCCUCUGA, SEQ ID NO: 13) is moved in the 5'-end direction in the nucleotide sequence of SEQ ID NO: 1. The nucleotide sequence of SEQ ID NO: 3 is a sequence, in which a part of guanines is substituted by uridines in the nucleotide sequence of SEQ ID NO: 1.

The nucleotide sequence of SEQ ID NO: 4 is a sequence, in which a part is substituted by consecutive uridines in the nucleotide sequence of SEQ ID NO: 1. The nucleotide sequences of SEQ ID NOS: 5, 6, 7, and 8 are each a sequence in which a part is substituted by an adenine-rich sequence in the nucleotide sequence of SEQ ID NO: 1. The nucleotide sequences of SEQ ID NOS: 9, 10, and 11 are a sequence, in which a part is arbitrarily substituted by another nucleotide in the nucleotide sequence of SEQ ID NO: 6.

The nucleotide sequence of SEQ ID NO: 12 is a sequence, in which at least one guanine is added in the nucleotide sequence of SEQ ID NO: 5.

Each nucleotide in nucleotide sequences of SEQ ID NOS: 1 to 12 may be modified from the naturally occurring form. For example, at least one U may be substituted by an N1-methyl-pseudouridine in the sequence. All Us may be substituted by N1-methyl-pseudouridines in the sequence.

### Example 2: 3'-UTR sequence increasing expression of gene encoding mRNA

A 3'-UTR sequence was designed that increases expression of a gene encoding an mRNA.

As a result, 7 3'-UTR sequences having nucleotide sequences of SEQ ID NOS: 14 to 20 were obtained.

The nucleotide sequence of SEQ ID NO: 14 may be a sequence not including a miRNA binding site. The nucleotide sequences of SEQ ID NOS: 15 to 18 may include a CU-rich element (UCCACCCCCCCAUCUCC, SEQ ID NO: 21).

The nucleotide sequences of SEQ ID NOS: 16 and 19 may include an antigen-R binding element (UUGGUUU).

Each nucleotide in nucleotide sequences of SEQ ID NOS: 14 to 20 may be modified from the naturally occurring form. For example, at least one U may be substituted by an N1-methyl-pseudouridine in the sequence. All Us may be substituted by N1-methyl-pseudouridines in the sequence.

### Example 3: Preparation of template vector using non-naturally occurring 5'-UTR sequence and 3'-UTR sequence

A vector was prepared by operatively linking: at least one sequence encoding non-naturally occurring 5'-UTR nucleotide sequence comprising nucleotide sequences of SEQ ID NOS: 1 to 12; at least one sequence encoding 3'-UTR nucleotide sequence comprising nucleotide sequences of SEQ ID NOS: 14 to 20; and a gene encoding a polypeptide.

Specifically, the vector may be one in which are connected: a promoter; a nucleotide sequence encoding the 5'-UTR; an open reading frame (ORF) encoding a polypeptide; a nucleotide sequence encoding the 3'-UTR; and a poly (A) tail. For the nucleotide sequence encoding the 5'-UTR, at least one selected from nucleotide sequences of SEQ ID NOS: 22 to 33 was used. For the nucleotide sequence encoding the 3'-UTR, at least one selected from nucleotide sequences of SEQ ID NOS: 109 to 115 was used. The nucleotide sequences of SEQ ID NOS: 109 to 115 correspond to nucleotide sequences of SEQ ID NOS: 34 to 40, comprising a recognition sequence for Xhol enzyme at its 5 terminus and a recognition sequence for Nhel enzyme at its 3 terminus.

The nucleotide sequence encoding the poly (A) tail has a nucleotide sequence of SEQ ID NO: 41. The vector may also include an E. coli replication origin ColE1 ori, and a selection marker gene. The promoter may be a T7 promoter. The selection marker may be a kanamycin resistance enzyme.

When the nucleotide sequence encoding the selected non-naturally occurring 5'-UTR and/or the nucleotide sequence encoding the 3'-UTR was linked to a sequence encoding a polypeptide, production of the polypeptide was increased, and stability of the mRNA, which includes the non-naturally occurring 5'-UTR sequence, the sequence encoding a polypeptide, and the 3'-UTR sequence, was significantly increased. The polypeptide may be a SARS-CoV-2 spike protein having each of amino acid sequences of SEQ ID NOS: 42 to 48, or alpha, beta, gamma, delta, epsilon, and omicron variants thereof. The polynucleotide encoding the SARS-CoV-2 spike protein or alpha, beta, gamma, delta, epsilon, and omicron variants thereof may have nucleotide sequences of SEQ ID NOS: 49 to 55, and the mRNA thereof may each have nucleotide sequences of SEQ ID NOS: 56 to 62. A cloning process of the mRNA encoding the polypeptide, for example, a SARS-CoV-2 spike protein, or a variant thereof is as follows.

### (1) Preparation of template vector for producing mRNA of SARS-CoV-2 spike protein or variant thereof

A vector for producing an mRNA of a SARS-CoV-2 spike protein or a variant thereof and/or a product of the protein was prepared, wherein the vector includes one of nucleotide sequences encoding the 12 designed 5'-UTR sequences, one of nucleotide sequences encoding a SARS-CoV-2 spike protein or a variant thereof, one of nucleotide sequences encoding the 7 3'-UTR sequences, and poly (A) tail, and the vector was introduced into cells of a microorganism, and the obtained cells of a microorganism was cultured to produce cloning vectors. Through in vitro transcription with the produced vector as a template, mRNA was produced. The variant includes alpha, beta, gamma, delta, epsilon, and omicron variants.

Specifically, a vector pUC57-Kan^{R}, in which an antibiotic resistance gene was substituted by a kanamycin resistance gene, was produced from the pUC57-Amp^{R} vector (Addgene). The produced pUC57-Kan^{R} vector was cleaved by treating with restriction enzymes Hindlll and EcoRl, and the cleaved vector was connected to a synthetically prepared polynucleotide encoding a SARS-CoV-2 spike protein or a variant thereof by using a ligase, and a pUC57-Kan^{R} vector having a sequence encoding a SARS-CoV-2 spike protein or a variant thereof inserted was obtained. The polynucleotide encoding a SARS-CoV-2 spike protein or a variant thereof has a nucleotide sequences of SEQ ID NOS: 49 to 55. The inserted polynucleotide encoding a spike protein or a variant thereof includes a nucleotide sequence encoding 5'-UTR sequence and a sequence encoding a SARS-CoV-2 spike protein or a variant thereof, and in the 879th nucleotide from the 5'-end of the nucleotide sequence encoding the SARS-CoV-2 spike protein is a BamHl restriction enzyme recognition sequence, and the recognition sequence was cleaved later to introduce nucleotide sequences encoding the designed 5'-UTR sequence.

### (2) Connection of nucleotide sequence encoding 5'-UTR sequence and nucleotide sequence encoding SARS-CoV-2 spike protein or variant thereof

In order to substitute the 5'-UTR sequence of the prepared template vector with a nucleotide sequence encoding a 5'-UTR sequence selected from sequences of SEQ ID NOS: 1 to 12, a DNA fragment containing the nucleotide sequence encoding the 5'-UTR was obtained through a PCR reaction with a primer set of a primer including the nucleotide sequence encoding the 5'-UTR and a primer including the sequence of the BamHl restriction enzyme recognition sequence of the SARS-CoV-2 spike protein, with the template vector as the template. In order to secure DNA fragments containing the nucleotide sequence encoding 5'-UTR more reliably, PCR reactions were carried out in two processes per a sequence. After carrying out a first PCR using a primary primer, a second PCR was performed using the obtained DNA as a template and using a secondary primer, and thus, DNA fragments containing the nucleotide sequence encoding 5'-UTR were obtained. The primer sequence used here was a polynucleotide of SEQ ID NOS: 77 to 100.

Each of DNA fragments containing nucleotide sequences encoding 12 5'-UTRs secured by PCR reaction, and the template vector prepared in (1) were treated with restriction enzymes Hindlll and BamHl to cleave, and the cleaved sequences were connected by using a ligase. As a result, vectors encoding the SARS-CoV-2 spike protein or variants thereof, in which the nucleotide sequence encoding 5'-UTR is substituted by each of nucleotide sequence encoding 5'-UTR selected from sequences of SEQ ID NOS: 1 to 12, were prepared.

### (3) Connection of nucleotide sequence encoding 3'-UTR

In the vectors prepared in (2), encoding a SARS-CoV-2 spike protein or a variant thereof, in which the nucleotide sequence encoding 5'-UTR is substituted by each nucleotide sequence encoding 5'-UTR selected from sequences of SEQ ID NOS: 22 to 33, each nucleotide sequence encoding 3'-UTR selected from sequences of SEQ ID NOS: 109 to 115 was connected to the 3'-end of the sequence encoding a SARS-CoV-2 spike protein or a variant thereof.

Each nucleotide sequences of SEQ ID NOS: 109 to 115 correspond to nucleotide sequences of SEQ ID NOS: 34 to 40, comprising a recognition sequence for Xhol enzyme at its 5 terminus and a recognition sequence for Nhel enzyme at its 3 terminus.

Specifically, each nucleotide sequence encoding 3'-UTR selected from sequences of SEQ ID NOS: 109 to 115, was synthetically secured. Each nucleotide sequence of SEQ ID NOS: 102 to 108 is RNA sequence corresponding to each nucleotide sequence of SEQ ID NOS: 109 to 115.

The secured sequence and the template vector prepared in (2) were cleaved by Xhol and Nhel restriction enzymes, and the products were connected by using a DNA ligase. As a result, a vector including a structure of a sequence encoding 5'-UTR-a sequence encoding a SARS-CoV-2 spike protein or a variant thereof-a sequence encoding 3'-UTR was prepared. Here, the sequence encoding 5'-UTR refers to each sequence encoding 5'-UTR sequence selected from sequences of SEQ ID NOS: 22 to 33. The sequence encoding 3'-UTR refers to each sequence encoding 3'-UTR sequence selected from sequences of SEQ ID NOS: 109 to 115. The sequence encoding a SARS-CoV-2 spike protein or a variant thereof indicates each amino acid sequence of each SARS-CoV-2 of SEQ ID NOS: 42 to 48 or a variant thereof.

### (4) Connection of consecutive adenine nucleotides

In the vector including the structure of a sequence encoding 5'-UTR-a sequence encoding a SARS-CoV-2 spike protein or a variant thereof-a sequence encoding 3'-UTR, a sequence of consecutive adenine nucleotides was connected to the 3'-end of the sequence encoding 3'-UTR.

A polyadenylate was synthetically obtained, in which Nhel and EcoRI sequences were respectively added to the 5'-end and the 3'-end in the sequence of consecutive adenylates of SEQ ID NO: 41. The secured sequence and the template vector were each cleaved by Nhel and EcoRI restriction enzymes, and the cleaved products were connected by using a DNA ligase.

As a result, a vector including a structure of a sequence encoding 5'-UTR-a sequence encoding a SARS-CoV-2 spike protein or a variant thereof- a sequence encoding 3'-UTR-poly (A) tail was prepared. Here, the sequence encoding 5'-UTR, the sequence encoding 3'-UTR, and sequence encoding a SARS-CoV-2 spike protein or a variant thereof are as described above. In addition, the poly (A) tail has a nucleotide sequence of SEQ ID NO: 41.

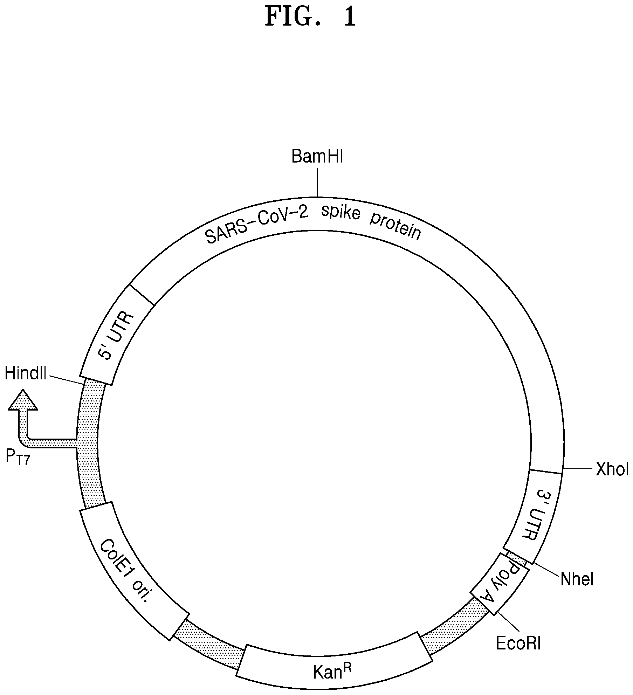

FIG. 1 shows a vector for producing an mRNA encoding a prepared SARS-CoV-2 spike protein, or a variant thereof.

### Example 4: Production of gene products using non-naturally occurring 5'-UTR sequence and 3'-UTR sequence

The vector prepared in Example 3, including a structure of a sequence encoding 5'-UTR-a sequence encoding a SARS-CoV-2 spike protein or a variant thereof- a sequence encoding 3'-UTR-poly (A) tail was introduced into E. coli to obtain transformed E. coli, and the obtained E.coli was cultured to proliferate E. coli containing the plasmid DNA, which is the template for an mRNA. The proliferated E. coli was isolated from the culture medium by high-speed centrifugation, and the cell-derived template DNA was secured by using the alkali extraction method known in the art. The secured template DNA was linearized by using the restriction enzyme recognition sequence located at the end of the poly (A) tail. mRNA having a nucleotide sequence of 5'-UTR-a sequence encoding a SARS-CoV-2 spike protein or a variant thereof-3'-UTR-poly (A) tail was produced by in vitro transcription using the linearized, cell-derived template DNA. Here, the sequence encoding the 5'-UTR, 3'-UTR, poly (A) tail, and SARS-CoV-2 spike protein or a variant thereof, are as described above.

Specifically, 5 U/µl of T7 RNA polymerase was added to an aqueous solution containing 10 ng/µl of linearized plasmid DNA, 4 mM of CleanCap^{®} Reagent AG, 5 mM of NTP, 5 mM of N1-methyl pseudouridine, 20 mM of magnesium ion salt, 10 mM of DTT, 0.01 U/µl of inorganic pyrophosphatase (PPase), and 1 U/µl of RNAse inhibitor, and the reaction mixture was incubated at 37 °C for at least one hour, and thus, an in vitro transcription reaction was proceeded. CleanCap^{®} Reagent AG is a reagent for mRNA transcription and co-transcriptional capping, and forms a cap structure of m7G(5')ppp(5')(2'OMeA)pG . The reaction products were treated with a DNAse, and RNA was purified by using a Monarch^{®} RNA Cleanup Kit (New England Biolabs). As a result, mRNA having a nucleotide sequences of SEQ ID NOS: 63 to 76 was produced. The 5'-UTR, the sequence of SARS-CoV-2 spike protein or a variant thereof, the 3'-UTR, and the nucleotide sequence of poly (A) tail included in the mRNA are as shown in Table 1 below.

**[Table 1]**

| SEQ ID NO | 5'-UTR sequence | Coding sequence of SARS-CoV-2 spike protein | 3'-UTR sequence | Poly (A) tail | Note |
|---|---|---|---|---|---|
| 63 | 1 | 56 | 108 | 101 | Wild type |
| 64 | 1 | 57 | 108 | 101 | Alpha |
| 65 | 1 | 58 | 108 | 101 | Beta |
| 66 | 1 | 59 | 108 | 101 | Gamma |
| 67 | 1 | 60 | 108 | 101 | Delta |
| 68 | 1 | 61 | 108 | 101 | Epsilon |
| 69 | 1 | 62 | 108 | 101 | Omicron |
| 70 | 10 | 56 | 108 | 101 | Wild type |
| 71 | 10 | 57 | 108 | 101 | Alpha |
| 72 | 10 | 58 | 108 | 101 | Beta |
| 73 | 10 | 59 | 108 | 101 | Gamma |
| 74 | 10 | 60 | 108 | 101 | Delta |
| 75 | 10 | 61 | 108 | 101 | Epsilon |
| 76 | 10 | 62 | 108 | 101 | Omicron |

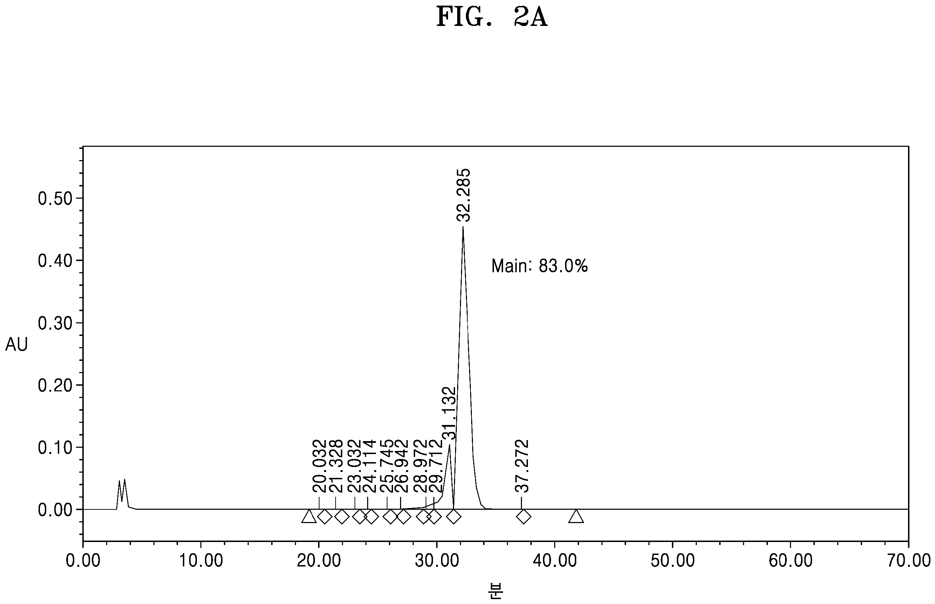

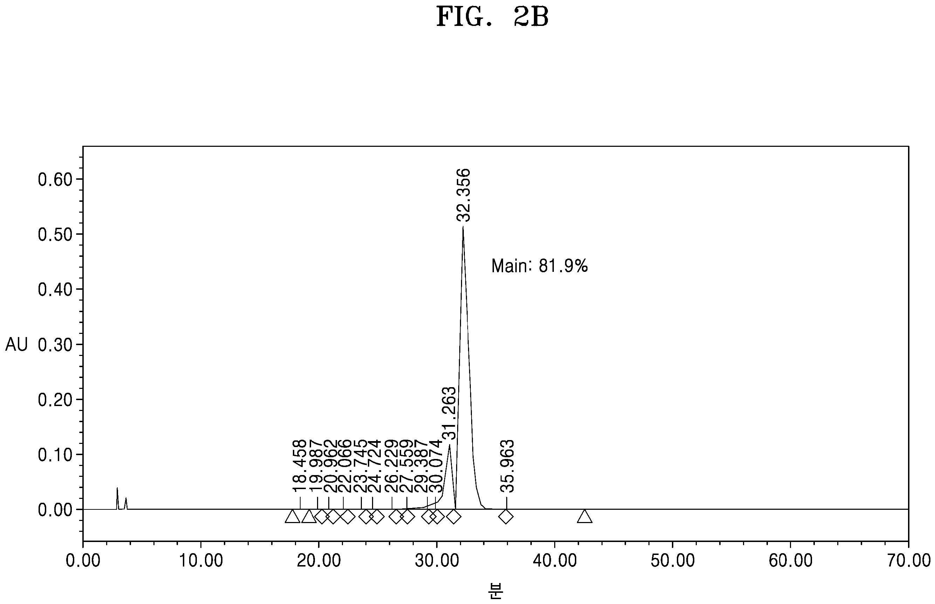

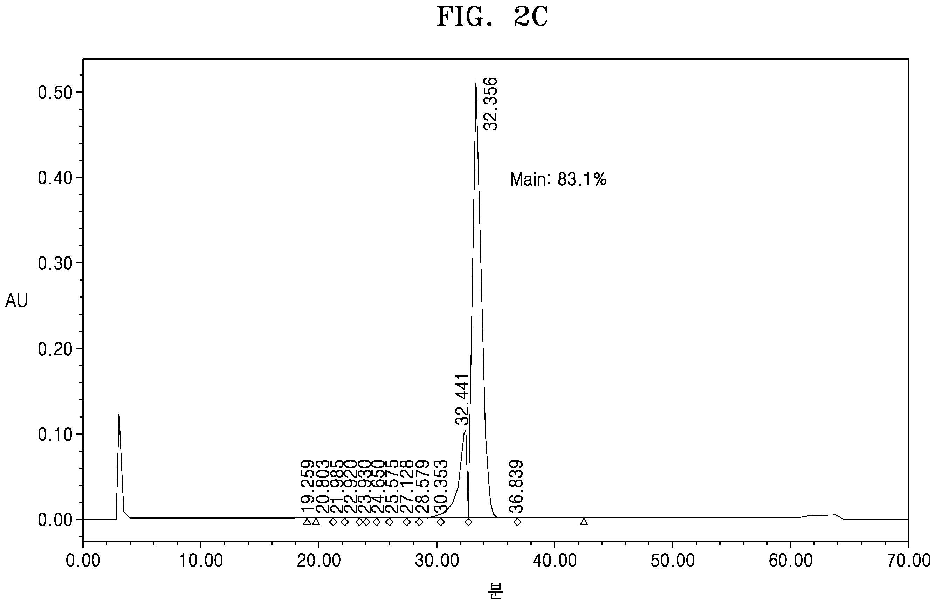

FIGS 2A, 2B, and 2C show results of chromatography analysis of mRNAs having a nucleotide sequence of SEQ ID NO: 67, 70, or 76 among the mRNAs produced. The biggest peaks in FIGS 2A, 2B, and 2C show mRNAs having a nucleotide sequence of SEQ ID NO: 67, 70, or 76, respectively. As shown in FIGS. 2A, 2B, and 2C, the purity of the mRNAs having a nucleotide sequence of SEQ ID NO: 67, 70, or 76 was 83.0 %, 81.9 % and 83.1 %, respectively.

### Example 5: Preparation of lipid nanoparticles loaded with gene product mRNA

ALC-0315, ((4-hydroxybutyl)azanediyl)bis(hexane-6,1-diyl)bis(2-hexyldecanoate)) (BLDpharm Ltd. China) as a ionizable lipid, (distearoylphosphatidylcholine, DSPC) (Avanti, USA) as a phospholipid, Synthechol (Sigma, USA) as a cholesterol, ALC-0159, 2-[(polyethylene glycol)-2000]-N,N-ditetradecylacetamide (SINOPEG, China) as a lipid-PEG conjugate were dissolved in ethanol at a molar ratio of 46.3 : 9.4 : 42.7 : 1.6, to prepare a lipid complex solution.

0.3 mg of the mRNA prepared in Example 4 was diluted to 0.08 mg/mL in 100 mM of sodium acetate, to obtain an mRNA-containing solution. The obtained mRNA solution and the lipid complex solution were injected at a flow rate of 12 mL/min into each microchannel of a microfluidic device (Ignite Nanoassemblr; PNI, Canada) at a volume ratio of 5 : 1 so that the weight ratio of the mRNA and the lipid complex may be 1 : 25.4, and thus, the mRNA solution and the lipid complex solution were mixed. By the mixing, the ethanol in the lipid complex solution was diluted to a critical ethanol concentration and lipid nanoparticles loaded with mRNA were formed. The microfluidic device includes a microchannel formed by convergence of two microchannels, and is a device that allows two solutions to mix while flowing, for example, by flowing an mRNA solution in a microchannel and flowing a lipid complex solution in another microchannel. The mixture eluted through the microchannel of the microfluidic device is an mRNA-loaded lipid nanoparticles (LNPs).

The prepared mRNA-loaded LNPs were loaded on a centrifugal filter of 10,000 Da and the buffer was exchanged with a buffer containing 0.45 mM of potassium chloride, 0.24 mM of monobasic potassium phosphate, 20.53 mM of sodium chloride, 1.31 mM of dibasic sodium phosphate and 2 % sucrose (pH 6.9 to pH 7.9). Thereafter, the mRNA-loaded LNP solution in the buffer was filtered through a 0.2 µm filter to prepare a final mRNA-loaded LNP.

### Example 6: Identification of properties of lipid nanoparticles

### (1) Identification of encapsulation efficiency of lipid nanoparticles

The loading or encapsulation efficiency (%) of the mRNA-loaded LNP (hereinafter also referred to as 'mRNA-LNP') prepared in Example 5 was identified by using a Quant-iT RiboGreen RNA Kit (Invitrogen, USA) according to the manufacturer's instructions. Specifically, Triton-X in the kit was mixed with the mRNA-loaded LNP to denature the LNP to release the mRNA. The mixed solution was added to the wells containing the Ribogreen reagent, and fluorescence emitted from the Ribogreen reagent was detected to detect the released mRNA. The encapsulation efficiency was calculated according to the following formula. Encapsulation efficienty (%) = (fluorescence of LNP treated with Triton X - fluorescence of LNP without Triton-X treatment) / (fluorescence of LNP treated with Triton X) x 100

**[Table 2]**

| mRNA-LNP | mRNA (SEQ ID NO) | Encapsulation efficiency (%) |
|---|---|---|
| Wildtype S | 70 | 92.8 |
| Delta S | 67 | 93.5 |
| Omicron S | 76 | 96.7 |

As shown in Table 2, the wildtype S, delta S, and omicron S mRNA-LNPs were identified to load mRNA with high encapsulation efficiency.

### (2) Identification of size of lipid nanoparticles

Particle sizes and polydispersity index (PDI) of the mRNA-LNPs prepared in Example 5 were identified by using Zetasizer NanoZS (Malvern Instruments, UK). Zetasizer is a device that determines particle size and particle size distribution by using dynamic light scattering technology. Table 3 shows results of measuring the particle size and polydispersity index.

**[Table 3]**

| mRNA-LNP | mRNA (SEQ ID NO) | Diameter (nm) | Polydispersity index |
|---|---|---|---|
| Wildtype S | 70 | 86.5 | 0.090 |
| Delta S | 67 | 81.4 | 0.096 |
| Omicron S | 76 | 96.7 | 0.024 |

As shown in Table 3, particle sizes of the wildtype S, delta S, and omicron S mRNA-LNPs were in a range of 80 nm to 100 nm.

### Example 7: Identification of mRNA expression in HEK293T cell

The mRNA-containing lipid nanoparticles prepared in Example 5 were delivered to HEK293T cells and mRNA expression was identified. To this end, an in vitro experiment was conducted as follows.

24 hours after seeding HEK293T cells in 6-well plates at 6×10⁵ cells/2 mL/well, 0.5 µg or 2 µg of each of the lipid nanoparticles was added and incubated for 24 hours. Culturing was performed at 37° C in a medium. After the culturing, 160 µl/well of mammalian protein extraction reagent (M-PER^{™}) (Thermo ScientificTM, Cat No. 78501), a lysis buffer, was added to the wells to rupture the cells and to extract the protein, and bicinchoninic acid assay (BCA) protein quantification was performed by using a Pierce^{™} BCA Protein Assay Kit (Thermo ScientificTM, Cat No. 23225). The obtained reagent was diluted by the lysis buffer and 5x reducing dye (10 % of SDS, 0.5 % of bromophenol blue, 50 % of glycerol, 0.5 % of 2-mercaptoethanol, 250 mM of tris of pH 6.8) so that protein concentrations may become equal. A western blot experiment was performed on the obtained diluted samples by using the anti-SARS-CoV-2 spike S1 subunit monoclonal antibody (R&D systems, Cat No. MAB105403).

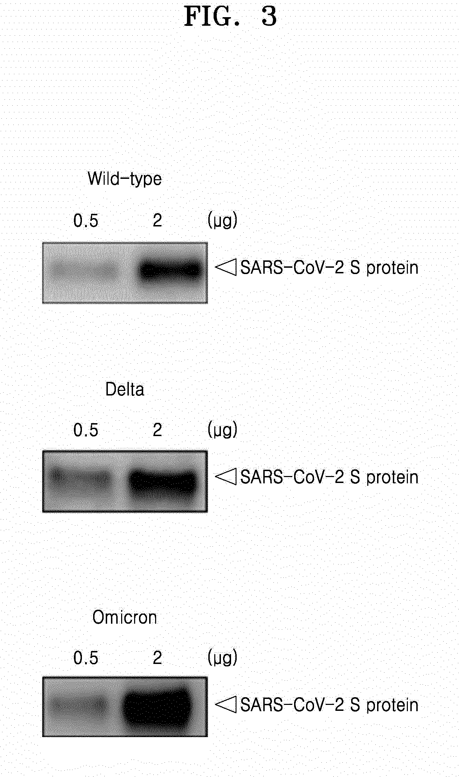

FIG. 3 shows results of identifying expression of the spike protein by using a western blot when the produced mRNA-lipid nanoparticles (LNPs) are introduced into a cell. As shown in FIG. 3, the expression of S protein increased as the concentration of mRNA-LNP treated increased.

## Claims

1. An isolated polynucleotide, which is a polydeoxyribonucleotide, comprising: a nucleotide sequence encoding a non-naturally occurring 5'-untranslated region (5'-UTR), a nucleotide sequence encoding a 3'-untranslated region (3'-UTR), or a combination thereof.

2. The polynucleotide of claim 1, wherein the 5'-UTR comprises a nucleotide sequence having sequence identity of 70 % or more to a nucleotide sequence of SEQ ID NO: 1.

3. The polynucleotide of claim 1, wherein the 3'-UTR comprises a nucleotide sequence having sequence identity of 80 % or more to a nucleotide sequence of SEQ ID NO: 15 or 20.

4. The polynucleotide of claim 1, wherein the nucleotide sequence encoding the 5'-UTR is selected from nucleotide sequences of SEQ ID NOS: 22 to 33.

5. The polynucleotide of claim 1, wherein the nucleotide sequence encoding the 3'-UTR is selected from the nucleotide sequences comprising each nucleotide sequence of SEQ ID NOS: 34 to 40.

6. The polynucleotide of claim 1, wherein the nucleotide sequence encoding the 5'-UTR further includes an upstream promoter region nucleotide sequence operatively linked to the 5'-UTR.

7. The polynucleotide of claim 1, wherein the nucleotide sequence encoding the 5'-UTR includes a transcribable nucleotide sequence or a nucleotide sequence for introducing the transcribable nucleotide sequence, and the transcribable nucleotide sequence or the nucleotide sequence for introducing the transcribable nucleotide sequence is operatively linked to the 5'-UTR, downstream of the nucleotide encoding the 5'-UTR.

8. The polynucleotide of claim 7, wherein the transcribable nucleotide sequence is a sequence encoding a polypeptide or RNA.

9. The polynucleotide of claim 8, wherein the polypeptide is an antigenic or therapeutic polypeptide.

10. The polynucleotide of claim 7, wherein the nucleotide sequence for introducing the transcribable nucleotide sequence is a cloning site.

11. The polynucleotide of any one of claims 7 to 10, wherein the transcribable nucleotide sequence or the nucleotide sequence for introducing the transcribable nucleotide sequence is operatively linked to the 3'-UTR, upstream of the nucleotide encoding the 3'-UTR, and
the nucleotide sequence encoding the 3'-UTR further comprises a nucleotide sequence of a downstream polyadenylate or a polyadenylate attachment signal (poly (A) attachment signal) operably linked to the 3'-UTR.

12. The polynucleotide of claim 11, wherein the nucleotide sequence encoding the 5'-UTR comprises a nucleotide sequence of SEQ ID NO: 22 or 31,
the nucleotide sequence encoding the 3'-UTR comprises a nucleotide sequence of SEQ ID NO: 40, and
the polyadenylate nucleotide sequence comprises a nucleotide sequence of SEQ ID NO: 41.

13. The polynucleotide of claim 1, wherein the nucleotide sequence encoding the 3'-UTR further comprises a nucleotide sequence of a downstream polyadenylate or a polyadenylate attachment signal (poly (A) attachment signal) operably linked to the 3'-UTR.

14. The polynucleotide of claim 1, wherein the nucleotide sequence encoding the 3'-UTR includes a transcribable nucleotide sequence or a nucleotide sequence for introducing the transcribable nucleotide sequence, and the transcribable nucleotide sequence or the nucleotide sequence for introducing the transcribable nucleotide sequence is operatively linked to the 3'-UTR, upstream of the nucleotide encoding the 3'-UTR.

15. The polynucleotide of claim 1, wherein the nucleotide sequence encoding the 5'-UTR comprises an upstream promoter region nucleotide sequence operatively linked to the 5'-UTR, a transcribable nucleotide sequence or a nucleotide sequence for introducing the transcribable nucleotide sequence operatively linked to the promoter; the transcribable nucleotide sequence or the nucleotide sequence for introducing the transcribable nucleotide sequence is operatively linked to a nucleotide sequence encoding the 5'-UTR, downstream of the nucleotide sequence encoding the 5'-UTR; and
the nucleotide sequence encoding the 3'-UTR comprises a nucleotide sequence of a downstream polyadenylate or a polyadenylate attachment signal operatively linked to the 3'-UTR, and is operatively linked to the transcribable nucleotide sequence or the nucleotide sequence for introducing the transcribable nucleotide sequence, upstream of the nucleotide sequence encoding the 3'-UTR.

16. The polynucleotide of claim 15, wherein the nucleotide sequence encoding the 5'-UTR is a nucleotide sequence of SEQ ID NO: 22 or SEQ ID NO: 31, and comprises a transcribable nucleotide sequence or a nucleotide sequence for introducing the transcribable nucleotide sequence operatively linked to the promoter; the transcribable nucleotide sequence or the nucleotide sequence for introducing the transcribable nucleotide sequence is operatively linked to the 5'-UTR sequence, downstream of the 5'-UTR sequence; and
the 3'-UTR comprises a nucleotide sequence of a downstream polyadenylate or a polyadenylate attachment signal operatively linked to the 3'-UTR, and is operatively linked to the transcribable nucleotide sequence or the nucleotide sequence for introducing the transcribable nucleotide sequence, upstream of the nucleotide sequence encoding the 3'-UTR.

17. The polynucleotide of any one of claims 1 to 16, wherein the polynucleotide is an expression construct or a vector.

18. RNA obtainable by transcription using a polynucleotide of any one of claims 1 to 16 as a template.

19. An isolated polynucleotide, which is a polyribonucleotide, comprising: a nucleotide sequence of a non-naturally occurring 5'-untranslated region (5'-UTR), a nucleotide sequence of a 3'-untranslated region (3'-UTR), or a combination thereof.

20. The polynucleotide of claim 19, wherein the 5'-UTR comprises a nucleotide sequence having sequence identity of 70 % or more to a nucleotide sequence of SEQ ID NO: 1.

21. The polynucleotide of claim 19, wherein the 3'-UTR comprises a nucleotide sequence having sequence identity of 80 % or more to a nucleotide sequence of SEQ ID NO: 15 or 20.

22. The polynucleotide of claim 19, wherein the 5'-UTR nucleotide sequence comprises a nucleotide sequence encoding a polypeptide, and the nucleotide sequence encoding a polypeptide is operatively linked to the 5'-UTR, downstream of the 5'-UTR nucleotide.

23. The polynucleotide of claim 22, wherein the polypeptide is an antigenic or therapeutic polypeptide.

24. The polynucleotide of claim 19, wherein the 3'-UTR nucleotide sequence further comprises a downstream polyadenylate nucleotide sequence operatively linked to the 3'-UTR.

25. The polynucleotide of claim 19, wherein the 3'-UTR nucleotide sequence comprises a nucleotide sequence encoding a polypeptide, and the nucleotide sequence encoding a polypeptide is operatively linked to the 3'-UTR, upstream of the 3'-UTR nucleotide.

26. The polynucleotide of claim 19, wherein the 5'-UTR nucleotide sequence comprises a nucleotide sequence encoding a polypeptide, and the nucleotide sequence encoding a polypeptide is operatively linked to the 5'-UTR, downstream of the 5'-UTR nucleotide, and
the 3'-UTR nucleotide sequence comprises a downstream polyadenylate nucleotide sequence operatively linked to the 3'-UTR, and is operatively linked to the nucleotide sequence encoding the polypeptide, upstream of the 3'-UTR nucleotide.

27. The polynucleotide of claim 26, wherein the 5'-UTR nucleotide sequence comprises a nucleotide sequence of SEQ ID NO: 1 or 10,
the 3'-UTR nucleotide sequence comprises a nucleotide sequence of SEQ ID NO: 20, and
the polyadenylate nucleotide sequence comprises a nucleotide sequence of SEQ ID NO: 101.

28. The polynucleotide of claim 27, wherein at least one U is substituted by an N1-methyl-pseudouridine in the polynucleotide.

29. The polynucleotide of claim 28, wherein the polynucleotide has any one nucleotide sequence selected from SEQ ID NOS: 63 to 76, and at least one U is substituted by an N1-methyl-pseudouridine in the polynucleotide.

30. The polynucleotide of claim 19, wherein the 5'-UTR or the 3'-UTR has an activity of increasing translation efficiency, stability of the nucleotide sequence encoding a polypeptide, or a combination thereof.

31. The polynucleotide of claim 19, wherein the 5'-end has a 5'-cap structure.

32. The polynucleotide of claim 19, wherein the polynucleotide comprises at least one modified nucleotide.

33. The polynucleotide of claim 32, wherein the modified nucleotide has at least one uridine substituted by an N1-methyl-pseudouridine.

34. A composition for delivering a polypeptide into a subject, wherein the composition comprises a polynucleotide of any one of claims 19 to 33 as an active ingredient.
